# EUROPEAN PATENT SPECIFICATION

(11) **EP 2 879 515 B1**
(45) Date of publication and mention of the grant of the patent: **18.05.2016**
(21) Application number: 13765416.6
(22) Date of filing: 30.07.2013
(51) Int. Cl.: A61K 36/02, A23L 3/48

(54) **METHOD AND INSTALLATION FOR MAKING SEAWEED MEAL**
VERFAHREN UND VORRICHTUNG ZUR HERSTELLUNG EINER ALGENMAHLZEIT
PROCÉDÉ ET INSTALLATION DE PRODUCTION DE FARINE D'ALGUES

(30) Priority: 03.08.2012 IT RM20120388
(43) Date of publication of application: 10.06.2015
(73) Proprietor: Algea AS, 6516 Kristiansund (NO)
(72) Inventor: DI TOMMASO, Donata, I-66030 Mozzagrogna (CH) (IT); DE FLAVIIS, Mauro, I-65015 Montesilvano (PE) (IT); GJERDE, Hege, Kristiansund N (NO)
(74) Representative: Bellomia, Paolo
(86) International application number: PCT/IB2013/001701
(87) International publication number: WO 2014/020423

(56) References cited:
- WO-A1-2009/051470
- GB-A- 2 408 045
- JP-A- 1 137 956
- JP-A- 6 062 809
- JP-A- 2000 236 851
- DATABASE WPI Week 201182 2011 Thomson Scientific, London, GB; AN 2011-P81917 XP002688674, & CN 102 228 247 A (WEIHAI JIAXIAO FOODSTUFF CO LTD) 2 November 2011 (2011-11-02)

## Description

### Technical field

This invention relates to a method and an installation for making seaweed meal, in particular for human consumption. The method and installation can be used in the field of the farming and food industry.

### Background art

In the prior art, a method for making seaweed meal (currently of the species "Ascophyllum nodosum") comprises firstly the step of cutting the seaweed on the sea floor. The cut seaweed is then left in its natural environment, the Norwegian sea, at a temperature throughout the entire year of between 0° and 5°C. The seaweed is then harvested using nets and stored on a ship for being transferred to a processing factory.

When it reaches the factory, the seaweed is processed immediately.

In the factory the seaweed passes through a chopping machine comprising two rollers having spikes which, rotating, force the seaweed into special cavities. In this way the length of the seaweed is reduced, in such a way that it can be fed inside a mill, which reduces the dimensions so as to increase the efficiency of the subsequent desiccating operations.

The desiccating occurs inside a rotary drum desiccator using a flow of hot and dry air at a temperature of 500-550 C necessary for the complete removal of all the water contained in the seaweed processed previously.

The time for passing through the desiccator by the seaweed, necessary for the above-mentioned complete removal of all the water contained in the seaweed, is approximately 15-20 minutes.

The desiccated seaweed is subsequently ground by a further mill.

The prior art process described above can be penalising, given the high temperatures involved in the desiccating step, for the active components present in the seaweed, especially for the antioxidant and polyphenolic component, as well as for the vitamin components.

### Disclosure of the invention

In this context, the technical purpose which forms the basis of this invention is to propose a method and an installation for making seaweed meal in such a way as to overcome the above mentioned disadvantages of the prior art.

More specifically, the aim of this invention is to provide a method and an installation for making seaweed meal which is able to preserve the organoleptic properties of the seaweed.

The technical purpose indicated and the aims specified are substantially achieved by a method and an installation for making seaweed meal comprising the technical features described in one or more of the appended claims.

### Brief description of the drawings

Further features and advantages of the present invention are more apparent from the non-limiting description which follows of a preferred, non-limiting embodiment of a method and an installation for making seaweed meal as illustrated in the accompanying drawings, in which:

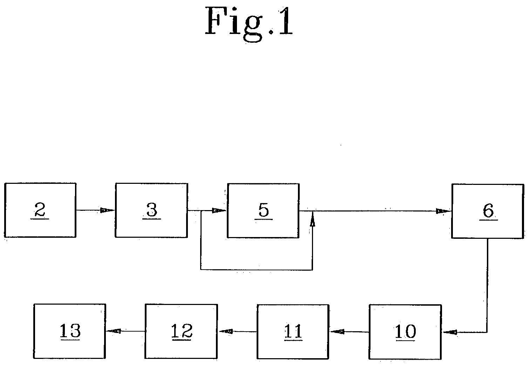

- Figure 1 is a block diagram of an installation for making seaweed meal according to this invention.

### Detailed description of the preferred embodiments of the invention

With reference to the accompanying drawing, the numeral 1 schematically indicates an installation for making seaweed meal according to this invention. The installation 1 is dedicated to the execution of the method for making seaweed meal according to this invention.

This method comprises, before the seaweed enters the installation 1, a step of harvesting the seaweed.

More specifically, the harvesting of the seaweed, preferably of the species "Ascophyllum nodosum", is performed by cutting. In more detail, the cutting is performed by using cutting bars, which are lowered into the water to a predetermined depth so as to remove only the apical part of the seaweed. The motion of the cutting bars is provided by suitable hydraulic movement means.

Advantageously, the harvesting by cutting, removing only the apical part of the seaweed, leaves the bush and the roots intact, allowing the subsequent regrowth. This regrowth occurs in the next three years allowing, as a further advantage, the same harvesting area to be used again after this period of time. Another advantage is due to the fact that this harvesting system, unlike the suction of the seaweed from the sea floor, guarantees a minimum or even zero presence of foreign bodies (sand, stones, etc.) in the raw material. Lastly, this method allows the complete regeneration of the entire ecosystem of the sea floor, and is therefore environmentally sustainable.

After the harvesting, the seaweed is transferred to the installation 1 for being processed. More in detail, the seaweed is unloaded and placed in bins, each of which is preferably able to contain approximately 500 kg. The bins are stored through a sliding door inside a dedicated refrigerating cell, made according to adequate heath standards.

The refrigerating cell has in particular an internal lining made from insulating panels preferably of polyurethane with concave mouldings. In more detail, the volume of the refrigerating cell is preferably 91 m³.

The refrigerating cell comprises a cooling system made preferably without the use of gas. The cooling system ensures a temperature of between 0°C and 6°C inside the refrigerating cell.

An evaporation system inside the refrigerating cell ensures the dehumidifying of the air inside the cell.

Advantageously, the storage under similar conditions considerably slows down the natural degradation of the seaweed, preserving the natural qualities for a longer time.

The processing of the seaweed proceeds with a chopping step, using a chopping device 2. For example, the chopping device 2 can comprise a pair of rollers equipped with spikes and facing each other. Moreover, cavities are made on the rollers. By rotating, the spikes of the rollers push the seaweed inside the cavities, so as to reduce the seaweed into pieces having a predetermined length.

After the chopping step, the pieces of seaweed are conveyed preferably by a conveyor belt into a cleaning device 3.

The cleaning device 3 comprises a further conveyor belt on which the seaweed is carried to the next apparatus. During transport the seaweed is struck by high pressure water coming from nozzles positioned above the conveyor belt so as to remove impurities and foreign bodies.

Advantageously, the cleaning step, performed by washing the seaweed in water, ensures the removal of the foreign bodies, including those small in size (such as, for example, sand). Moreover, the system subjects the seaweed to a low mechanical stress, contributing towards keeping unchanged the organoleptic properties of the seaweed.

The seaweed is then conveyed towards a desalination/deionization tank 5 or, alternatively, to the draining.

More specifically, the desalination tank 5 allows the content of salt and iodine in the final product to be reduced, if that is requested.

In more detail, following the cleaning, a mesh conveyor keeps the seaweed immersed inside the desalination tank 5. In more detail, the mesh conveyor belt immerses at an immersing side of the desalination tank 5. Moreover, the mesh conveyor belt emerges at an emerging side opposite the immersing side.

In further detail, the desalination tank 5 contains approximately 15 m3 of drinking water, which is constantly re-integrated in such a way as to maintain a difference in the saline concentration between the seaweed and the water. The water in excess is discharged through an opening positioned at a predetermined height.

The natural process of osmosis is accelerated by the constant movement of the water guaranteed by a circulating device with a high capacity of 2,000 litres per minute, agitating the water and, consequently, increasing the exchange between the water and the seaweed which is immersed. The water is drawn from the top of the desalination tank and reintroduced into the tank from above thorough sprinklers longitudinal with respect to the tank.

Lastly, at the emerging side a group of nozzles sprays the seaweed with low pressure water. In this way, the seaweed undergoes a last rinse with non-iodized water and, also, the water inside the desalination tank 5 is topped up.

The desalination tank 5 preferably has a desalinating/deionizing capacity equal to approximately 3,000 kg of seaweed in 4-6 hours. In more detail, the time of passing through the desalination tank 5 can vary depending on the quality of the seaweed introduced and the desired values of residual salt and iodine.

The seaweed at the outfeed from the desalination tank 5, or directly after the cleaning using a by-pass conveyor belt, is fed to the agitator means 6.

The means 6 for agitating the seaweed have the function of draining the seaweed and drying it of the surface washing water before the desiccating. The agitator means 6 act at low speed and at high pressure, in such a way as to remove the surface water from the seaweed.

More specifically, the drying is performed in a screw conveyor with a perforated casing with introduction of compressed air at a speed of rotation between 5 rpm and 60 rpm, preferably 15 rpm.

In more detail, the drying system comprises a rotary screw conveyor contained in a fixed cylinder. The cylinder has a surface having a plurality of slots in the first section and holes in the second section. Advantageously, the excess surface water is removed by mechanical action by the mixing generated by the rotation of the screw conveyor and by the particular internal shape of the cylinder. Furthermore, ventilation means, for example nozzles, are associated with the cylinder. Consequently, a flow of low pressure air strikes the inside of the cylinder, and in particular the seaweed located inside. The flow of air in the first section is greater than in the second section whilst the pressure is less in the first section and greater in the second section. In other words, during the drying step the seaweed is agitated and, at the same time, struck by the low pressure flow of air. This contributes towards making the draining more accurate. In further detail, the seaweed is struck transversely with a flow of air having a pressure of between 1 bar and 6 bar, and also having a temperature preferably between 5°C and 20°C.

The cylinder has an infeed side and an outfeed side opposite the infeed side. More specifically, the outfeed side is positioned higher than the infeed side. In other words, the cylinder is positioned in such a way that the relative axis of rotation is significantly inclined towards the infeed side, opposing in this way the forward movement of the seaweed.

According to the preferred embodiment of the invention, the agitator means 6 have a capacity of approximately 800-900 kg/h.

Advantageously, the low speed of rotation and the low pressure of the air blown inside the cylinder limit the stress of the seaweed, thus guaranteeing the preservation of its organoleptic properties.

A mincer 10 enters into operation on the seaweed at the outfeed of the agitator means 6, in particular from the cylinder.

More specifically, the mincing of the seaweed occurs between the drying step and a subsequent desiccating step. This mincing step is accompanied by a step for dehydrating the seaweed, performed in a vacuum and at a low temperature, more specifically below 60°C.

In more detail, the mincer 10 comprises a drum (not illustrated), preferably having a capacity of 250 litres. The drum has an upper opening through which the seaweed is inserted. The upper opening can be closed by a cover. A knife is installed at the bottom of the drum, equipped with a pair of blades opposite each other. The knife is rotated by movement associated with it.

The above-mentioned dehydrating step comprises a sub-step of heating the seaweed by passing a warming agent through the gap in the drum. In more detail, the gap in the drum is designed to allow the passage of steam or water, for heating or cooling, respectively, the contents of the drum. A water ring vacuum pump allows the vacuum to be created inside the drum.

During the mincing the seaweed is heated by low pressure steam, preferably between 0.5 bar and 2 bar. More specifically, the steam is introduced into the above-mentioned gap. Due to the vacuum created in the drum the seaweed is dehydrated without ever exceeding the temperature of 60°C.

Advantageously, the organoleptic properties of the seaweed are in this way further preserved.

The minced seaweed then continues through the desiccator 11. The desiccator comprises a drum, having an infeed side and an outfeed side for the seaweed. The drum also has an inner surface equipped with a plurality of blades. It should be noted that, according to this invention, the drum is inclined and can rotate about an axis of rotation with a variable speed.

The seaweed moves forward along the drum and, whilst it is inside, it is struck by a current of hot air which proceeds along the same direction as the seaweed, that is to say, in co-current flow.

More specifically, an LPG burner located at the infeed side of the drum has the purpose of heating the air. The air heated in this way exchanges heat when it encounters the seaweed, which is continuously stirred by the rotation of the drum. The air at the outfeed from the drum is removed by a fan, and is also filtered in order to order to avoid any dispersion of fine dusts in suspension into the environment.

It should be noted that the feeding of seaweed finely minced and already partly dehydrated allows the temperature of the seaweed at the outfeed from the desiccator 11 to be maintained at not more than 60°C, which corresponds to a temperature of the infeed air of between 150°C and 300°C. The air at the outfeed from the desiccator 11 has a temperature variable between 80°C and 100°C.

According to this invention, the seaweed passes through the drum in a time which can vary between 5 and 12 minutes.

This advantageously allows in particular the antioxidant components present in the seaweed, such as, for example, phlorotannins, polyphenols and vitamins, to be preserved.

The desiccator 11 has a production capacity of between 150 and 200 kg/h.

The seaweed then continues towards the grinder 12. More specifically, the desiccated seaweed passes through a selector valve, which diverts a flow of seaweed between a main storage and an alternative storage. More specifically, if the main storage is too full or if there are interruptions in the operation of the grinder 12, build-up or saturation of the installation can easily be avoided in such a way as to facilitate the dry cleaning. Advantageously, in this way it is possible to guarantee improved hygiene conditions in the installation 1.

During this step, the seaweed passes in front of magnet, which has the purpose of capturing any metallic particles, and in front of a metal detector, which has the purpose of automatically rejecting any seaweed which has been polluted.

The minced seaweed is fed to the grinder 12, which actually produces the meal, in particular through a relative lower zone. Subsequently, the meal is drawn by a fan into an upper area of the grinder 12. Advantageously, the continuous flow of air used as eluent considerably reduces the increase in temperature inside the grinder 12 and, consequently, of the meal.

The desired particle size of the finished product is obtained by adjusting the speed of a selector inserted in the grinder 12.

Advantageously, the reduced size of the particles at the outfeed of the desiccator 11 and their low temperature allows the use of a single grinding mill. This allows the accumulation points of the product to be reduced, in such a way as to further improve the hygiene conditions of the installation 1.

As a further advantage, there is a drastic limitation in the mechanical and thermal stress due, respectively, to the grinding and the further heating of the product, thereby allowing the organoleptic properties of the seaweed to be preserved.

At the outfeed from the grinder the seaweed meal is directed to a filler 13.

An operator adjusts the quantity of meal to be filled, setting the number of revolutions in a dosing screw conveyor, and gives the command to start the filling step after having positioned an empty bag in a special housing.

After filling the bag, the operator closes the bag by heat sealing it and, after it has been packaged, stores the bag in the store.

## Claims

1. A method for making seaweed meal, comprising the steps of harvesting the seaweed from a sea floor; chopping the seaweed; cleaning the seaweed; desiccating the seaweed; grinding the seaweed to a meal; **characterized in that** it comprises a drying step between the cleaning step and the desiccating step, the drying step being accomplished by seaweed agitator means (6), the agitator means (6) operating at low speed and low pressure.

2. The method for making seaweed meal according to the preceding claim, **characterized in that** the drying step comprises the sub-step of agitating the seaweed and at the same time blowing air through them transversely at a pressure between 1 bar and 6 bar, and at a temperature preferably between 5°C and 20°C.

3. The method for making seaweed meal according to the preceding claim, **characterized in that** the drying step is performed in a screw conveyor with a cylindrical casing (7) with a perforated surface and turning at a speed of rotation between 5 rpm and 60 rpm, preferably 15 rpm.

4. The method according to any of the preceding claims, **characterized in that** it comprises a step of mincing the seaweed between the drying step and the desiccating step and accompanied by a step of dehydrating the seaweed, the dehydrating step being performed in a vacuum and at a low temperature, preferably below 60°C.

5. The method for making seaweed meal according to the preceding claim, **characterized in that** the mincing step is accomplished by placing the seaweed in a drum comprising mincing means, preferably rotary blades, the dehydrating step comprising a sub-step of warming the seaweed by passing a warming agent through a gap in the drum and simultaneously connecting the inside of the drum to a pump to reduce the pressure and create a vacuum.

6. The method for making seaweed meal according to any of the preceding claims, **characterized in that** it comprises a step of desalinating the seaweed, the desalinating step being accomplished by immersing the seaweed, supported on a mesh conveyor belt, in a desalination tank (5) and causing a large quantity of water to circulate in order to generate a seaweed mixing motion while the seaweed is supported by the mesh conveyor belt.

7. The method for making seaweed meal according to any of the preceding claims, **characterized in that** the step of desiccating the seaweed is accomplished by blowing air through them at an inflow temperature between 150° and 300°C.

8. The method for making seaweed meal according to any of the preceding claims, **characterized in that** the desiccating step has a duration of between 7 and 10 minutes.

9. An installation (1) for making seaweed meal, comprising a seaweed chopper (2); a seaweed desiccator (11) located downstream of the chopper (2); a seaweed grinder (12) for obtaining the meal, located downstream of the chopper (2); **characterized in that** it comprises seaweed agitator means (6), located upstream of the dessicator and configured in such a way as to blow air through the seaweed at low pressure in order to reduce the water content of the seaweed.

10. The installation (1) for making seaweed meal according to the preceding claim, **characterized in that** the agitator means (6) comprise a cylinder with a perforated surface and housing a screw conveyor which turns at a speed of rotation between 5 rpm and 60 rpm, preferably 15 rpm.

## Patentansprüche

1. Verfahren zur Herstellung eines Seealgenmehls, umfassend die Schritte zum Ernten der Algen von einem Meeresboden, Hacken der Algen, Reinigen der Algen, Dörren der Algen, Mahlen der Algen zu einem Mehl, **dadurch gekennzeichnet, dass** es einen Schritt zum Trocknen zwischen dem Schritt zum Reinigen und dem Schritt zum Dörren umfasst, wobei der Schritt zum Trocknen durch Algenrüttelmittel (6) ausgeführt wird, wobei die Rüttelmittel (6) bei niedriger Geschwindigkeit und niedrigem Druck arbeiten.

2. Verfahren zur Herstellung eines Seealgenmehls nach dem vorhergehenden Anspruch, **dadurch gekennzeichnet, dass** der Schritt zum Trocknen den Unterschritt zum Rütteln der Algen und zum gleichzeitigen Blasen von Luft quer durch diese bei einem Druck von 1 bis 6 bar und bei einer Temperatur, die vorzugsweise zwischen 5 °C und 20 °C beträgt, umfasst.

3. Verfahren zur Herstellung eines Seealgenmehls nach dem vorhergehenden Anspruch, **dadurch gekennzeichnet, dass** der Schritt zum Trocknen in einem Schraubenförderer mit einem zylindrischen Gehäuse (7) mit einer gelochten Oberfläche erfolgt, der bei einer Drehzahl von 5 bis 60 U/min, vorzugsweise 15 U/min, verfährt.

4. Verfahren nach einem der vorhergehenden Ansprüche, **dadurch gekennzeichnet, dass** es einen Schritt zum Zerkleinern der Algen zwischen dem Schritt zum Trocknen und dem Schritt zum Dörren umfasst, der durch einen Schritt zum Dehydrieren der Algen umfasst, wobei der Schritt zum Dehydrieren in einem Vakuum und bei niedriger Temperatur, vorzugsweise unter 60 °C, durchgeführt wird.

5. Verfahren zur Herstellung eines Seealgenmehls nach dem vorhergehenden Anspruch, **dadurch gekennzeichnet, dass** der Schritt zum Zerkleinern erfolgt, indem die Algen in einer Trommel platziert werden, die Zerkleinerungsmittel, vorzugsweise rotierende Messer, umfasst, wobei der Schritt zum Dehydrieren einen Unterschritt zum Erwärmen der Algen umfasst, indem diese durch einen Spalt in der Trommel ein Wärmungsmittel passieren, wobei gleichzeitig der Innenraum der Trommel mit einer Pumpe verbunden wird, um den Druck zu reduzieren und ein Vakuum zu erzeugen.

6. Verfahren zur Herstellung eines Seealgenmehls nach einem der vorhergehenden Ansprüche, **dadurch gekennzeichnet, dass** es einen Schritt zum Entsalzen der Algen umfasst, wobei der Schritt zum Entsalzen erfolgt, indem die Algen, getragen auf einem Gitterförderband, in einen Entsalzungstank (5) getaucht werden, und dafür gesorgt wird, dass eine große Menge an Wasser zirkuliert, um eine Algenrührbewegung zu erzeugen, während die Algen vom Gitterförderband getragen werden.

7. Verfahren zur Herstellung eines Seealgenmehls nach einem der vorhergehenden Ansprüche, **dadurch gekennzeichnet, dass** der Schritt zum Trocknen der Algen erfolgt, indem Luft durch diese bei einer Einströmungstemperatur von 150° bis 300 °C geblasen wird.

8. Verfahren zur Herstellung eines Seealgenmehls nach einem der vorhergehenden Ansprüche, **dadurch gekennzeichnet, dass** der Schritt zum Dörren eine Dauer von 7 bis 10 Minuten aufweist.

9. Anlage (1) zur Herstellung eines Seealgenmehls, umfassend ein Algenhackmesser (2), eine Algenexsikkator (11), der nach dem Hackmesser (2) angeordnet ist, ein Algenmahlwerk (12) zum Erhalten des Mehls, das nach dem Hackmesser (2) angeordnet ist, **dadurch gekennzeichnet, dass** sie Algenrüttelmittel (6) umfasst, die vor dem Exsikkator angeordnet und ausgelegt sind, sodass Luft bei niedrigem Druck durch die Algen geblasen wird, um den Wassergehalt in den Algen zu reduzieren.

10. Anlage (1) zur Herstellung eines Seealgenmehls nach dem vorhergehenden Anspruch, **dadurch gekennzeichnet, dass** die Rüttelmittel (6) einen Zylinder mit einer gelochten Oberfläche umfassen und einen Schraubenförderer aufnehmen, der bei einer Drehzahl zwischen 5 und 60 U/min, vorzugsweise 15 U/min, verfährt.

## Revendications

1. Procédé de production de farine d'algues, comprenant les étapes consistant à récolter les algues sur le fond de la mer ; à broyer les algues ; à nettoyer les algues ; à dessécher les algues ; à réduire en farine les algues ; **caractérisé en ce qu'**il comprend une étape de séchage entre l'étape de nettoyage et l'étape de dessiccation, l'étape de séchage étant effectuée par des moyens agitateurs d'algues (6), les moyens agitateurs (6) fonctionnant à vitesse lente et à faible pression.

2. Procédé de production de farine d'algues selon la revendication précédente, **caractérisé en ce que** l'étape de séchage comprend une sous-étape consistant à agiter les algues et en même temps à y insuffler de l'air de façon transversale à une pression comprise entre 1 et 6 bars et à une température comprise de préférence entre 5 et 20 °C.

3. Procédé de production de farine d'algues selon la revendication précédente, **caractérisé en ce que** l'étape de séchage est effectuée dans un transporteur à vis doté d'une enveloppe cylindrique (7) ayant une surface perforée et tournant à une vitesse de rotation comprise entre 5 et 60 t/mn, de préférence 15 t/mn.

4. Procédé selon l'une quelconque des revendications précédentes, **caractérisé en ce qu'**il comprend une étape consistant à émincer les algues entre l'étape de séchage et l'étape de dessiccation, accompagnée d'une étape consistant à déshydrater les algues, l'étape consistant à déshydrater étant effectuée dans un vide à basse température, de préférence en dessous de 60 °C.

5. Procédé de production de farine d'algues selon la revendication précédente, **caractérisé en ce que** l'étape consistant à émincer est effectuée en plaçant les algues dans un tambour comprenant des moyens à émincer, de préférence des lames rotatives, l'étape de déshydratation comprenant une sous-étape de réchauffage des algues en passant un agent de réchauffage à travers un interstice dans le tambour et reliant simultanément l'intérieur du tambour à une pompe pour réduire la pression et créer un vide.

6. Procédé de production de farine d'algues selon l'une quelconque des revendications précédentes, **caractérisé en ce qu'**il comprend une étape de désalinisation des algues, l'étape de désalinisation étant effectuée en plongeant les algues, supportées sur un tapis transporteur métallique, dans un réservoir de désalinisation (5) pour faire en sorte qu'une grande quantité d'eau circule afin de générer un déplacement du mélange d'algues pendant que les algues sont supportées par le tapis transporteur métallique.

7. Procédé de production de farine d'algues selon l'une quelconque des revendications précédentes, **caractérisé en ce que** l'étape de dessiccation des algues est effectuée en y insufflant de l'air à une température d'entrée comprise entre 150 et 300 °C.

8. Procédé de production de farine d'algues selon l'une quelconque des revendications précédentes, **caractérisé en ce que** la durée de l'étape de dessiccation est comprise entre 7 et 10 minutes.

9. Installation (1) de production de farine d'algues, comprenant un broyeur d'algues (2) ; un dessiccateur d'algues (11) situé en aval du broyeur (2) ; une machine à meuler les algues (12), pour obtenir de la farine, située en aval du broyeur (2) ; **caractérisée en ce qu'**elle comprend des moyens agitateurs d'algues (6) situés en amont du dessiccateur et configurés de manière à insuffler de l'air dans les algues à basse pression afin de réduire l'eau contenue dans les algues.

10. Installation (1) de production de farine d'algues selon la revendication précédente, **caractérisée en ce que** les moyens agitateurs (6) comprennent un cylindre ayant une surface perforée et logeant un transporteur à vis tournant à une vitesse de rotation comprise entre 5 et 60 t/mn, de préférence 15 t/mn.
